(19) **Europäisches Patentamt**
**European Patent Office**
**Office européen des brevets**

(11) **EP 4 586 156 A1**

(12) **EUROPEAN PATENT APPLICATION**

(43) Date of publication:
**16.07.2025 Bulletin 2025/29**

(21) Application number: **25150727.3**

(22) Date of filing: **08.01.2025**

(51) International Patent Classification (IPC):
***G06N 20/00*** (2019.01)     ***G06N 7/01*** (2023.01)
***G06N 5/045*** (2023.01)

(52) Cooperative Patent Classification (CPC):
**G06N 20/00; G06N 7/01;** G06N 5/045

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.01.2024 JP 2024001892**

(71) Applicant: **Canon Medical Systems Corporation
Tochigi 324-0036 (JP)**

(72) Inventors:
• **ARITA, Kosuke**
**Otawara-shi, 324-0036 (JP)**
• **SASAKI, Sho**
**Otawara-shi, 324-0036 (JP)**
• **MATSUMURA, Yuka**
**Otawara-shi, 324-0036 (JP)**
• **MURAGUCHI, Yohei**
**Otawara-shi, 324-0036 (JP)**

(74) Representative: **Marks & Clerk LLP
15 Fetter Lane
London EC4A 1BW (GB)**

(54) **MEDICAL INFORMATION PROCESSING DEVICE, MEDICAL INFORMATION PROCESSING METHOD, AND PROGRAM**

(57)     A medical information processing device of an embodiment includes an acquirer, an inference basis determiner, a concept reflection degree calculator, and a dependency determiner. The acquirer is configured to acquire a machine learning model and training data used to train the machine learning model. The inference basis determiner is configured to determine an inference basis for each piece of the training data using the machine learning model to generate inference basis visualization results. The concept reflection degree calculator is configured to determine a concept emphasized by the machine learning model during inference based on the inference basis visualization results and calculate a concept reflection degree of each piece of the training data related to the concept. The dependency determiner is configured to generate visualization information of a dependency between the concept and a feature interpretable by a user in the training data based on the concept reflection degree and the feature interpretable by the user.

*FIG. 1*

**Description**

FIELD

Embodiments disclosed in this specification and drawings relate to a medical information processing device, a medical information processing method, and a program.

BACKGROUND

In training a machine learning model, learning may be performed using artifacts or biases (hereinafter referred to as "confounding factors") that are unrelated to class classification as clues. When learning is performed using such confounding factors as clues, inference is made on the basis of false correlations, resulting in a model with low accuracy for unknown data of a type that was not included in training data.

As a method of performing training without being affected by confounding factors, a method has been proposed in which images that are highly similar to an image designated by a user are extracted from medical images and images suitable for use as training data are selected. However, this method requires excluding images containing confounding factors, and therefore, when applied to a small amount of training data, the number of pieces of data is excessively reduced, and there is a risk of a model having low accuracy for unknown data.

Further, as a method of performing training robustly against confounding factors without reducing the amount of training data, a method has been proposed in which a model is updated such that the inference basis does not emphasize information on confounding factors, but emphasizes clinically valid information (hereinafter referred to as "clinical concepts"). However, this method requires doctors to manually label a clinical concept for each piece of training data, which places a heavy burden on doctors. As an alternative method, it is possible to identify and label clinical concepts with unsupervised learning by clustering the inference basis. However, it is not easy to check whether the inference basis of a trained machine learning model matches clinical concepts. This may result in learning that places emphasis on incorrect clinical concepts.

The problem to be solved by embodiments disclosed in this specification and the drawings is to confirm clinical validity of inference basis of a machine learning model. However, the problem to be solved by the embodiments disclosed in this specification and the drawings are not limited to the above problem. Problems corresponding to the effects of each configuration shown in the embodiments described later can also be positioned as other problems.

A medical information processing device of an embodiment includes an acquirer, an inference basis determiner, a concept reflection degree calculator, and a dependency determiner. The acquirer is configured to acquire a machine learning model and training data used to train the machine learning model. The inference basis determiner is configured to determine an inference basis for each piece of the training data using the machine learning model to generate inference basis visualization results. The concept reflection degree calculator is configured to determine a concept emphasized by the machine learning model during inference based on the inference basis visualization results and calculate a concept reflection degree of each piece of the training data related to the concept. The dependency determiner is configured to generate visualization information of a dependency between the concept and a feature interpretable by a user in the training data based on the concept reflection degree and the feature interpretable by the user.

The medical information processing device may further include an editor configured to edit the visualization information in response to an instruction from the user, and a model updater configured to update the machine learning model based on the edited visualization information.

The dependency determiner may be configured to generate a dependency graph, which is the visualization information, by graphical modeling.

The medical information processing device may further include a display controller configured to cause a display device to display the visualization information.

The model updater may be configured to additionally train the machine learning model to match the dependency derived from the machine learning model with a dependency corresponding to the edited visualization information.

The concept reflection degree calculator may be configured to determine the concept by clustering the inference basis visualization results based on a similarity of the inference basis visualization results, and calculate the concept reflection degree of each piece of the training data based on a distance from a cluster centroid of the clustered concept.

The concept reflection degree calculator may be configured to calculate the concept reflection degree such that the concept reflection degree increases as the distance from the cluster centroid of the clustered concept decreases and decreases as the distance increases.

When the training data is image data, the feature interpretable by the user may include at least one of a feature with respect to a color, a feature with respect to texture, and a feature with respect to a shape.

When the training data is non-image data, the feature interpretable by the user may include features calculated based on a predetermined guideline.

The editor may be configured to add the interpretable feature designated by the user to the visualization information.

The editor may be configured to delete the interpretable feature designated by the user from the visualization information.

The editor may be configured to newly add a definition of the interpretable feature based on an instruction from the user.

The feature interpretable by the user may be predefined.

A medical information processing method of another embodiment includes, using a computer, acquiring a machine learning model and training data used to train the machine learning model, determining an inference basis for each piece of the training data using the machine learning model to generate inference basis visualization results, determining a concept emphasized by the machine learning model during inference based on the inference basis visualization results and calculating a concept reflection degree of each piece of the training data related to the concept, and generating visualization information of a dependency between the concept and a feature interpretable by a user in the training data based on the concept reflection degree and the feature interpretable by the user.

A program of another embodiment causes a computer to acquire a machine learning model and training data used to train the machine learning model, determine an inference basis for each piece of the training data using the machine learning model to generate inference basis visualization results, determine a concept emphasized by the machine learning model during inference based on the inference basis visualization results and calculate a concept reflection degree of each piece of the training data related to the concept; and generate visualization information of a dependency between the concept and a feature interpretable by a user in the training data based on the concept reflection degree and the feature interpretable by the user.

BRIEF DESCRIPTION OF THE DRAWINGS

FIG. 1 is a functional block diagram showing an example of a configuration of a medical information processing device 1 according to an embodiment.

FIG. 2 is a flowchart showing an example of processing of the medical information processing device 1 according to the embodiment.

FIG. 3 is a diagram showing an example of a concept clustering result CR in a machine learning model according to the embodiment.

FIG. 4 is a diagram showing an example of a concept reflection degree RG for each piece of training clinical data according to the embodiment.

FIG. 5 is a diagram showing an example of a dependency graph DG according to the embodiment.

FIG. 6 is a diagram showing an example of a first screen PG1 displayed on a terminal device D according to the embodiment.

FIG. 7 is a diagram showing an example of the first screen PG1 and a second screen PG2 (when a node is added) displayed on the terminal device D according to the embodiment.

FIG. 8 is a diagram showing an example of a third screen PG3 (when a node is added) displayed on the terminal device D according to the embodiment.

FIG. 9 is a diagram showing an example of the first screen PG1 and a fourth screen PG4 (when a node is deleted) displayed on the terminal device D according to the embodiment.

FIG. 10 is a diagram showing an example of the first screen PG1 and a fifth screen PG5 (during edge editing) displayed on the terminal device D according to the embodiment.

FIG. 11 is a diagram showing an example of a sixth screen PG6 (during model updating) displayed on the terminal device D according to the embodiment.

FIG. 12 is a diagram showing an example of a flow of model update processing according to the embodiment.

DETAILED DESCRIPTION

Hereinafter, a medical information processing device, a medical information processing method, and a program according to an embodiment will be described with reference to the drawings. The medical information processing device according to the embodiment provides information indicating a relationship (dependency) between information (hereinafter referred to as a "concept") emphasized by a machine learning model during inference and features calculated from training data, thereby making it possible to confirm the clinical validity of the inference basis of the machine learning model. Furthermore, by updating the machine learning model to match the relationship edited according to instructions of an operator (doctor, etc.), it is possible to generate a machine learning model capable of operating with an inference basis corresponding to the intuition (clinical knowledge) of a doctor, and thus improve the accuracy of the machine learning model.

[Configuration of Medical Information Processing Device]

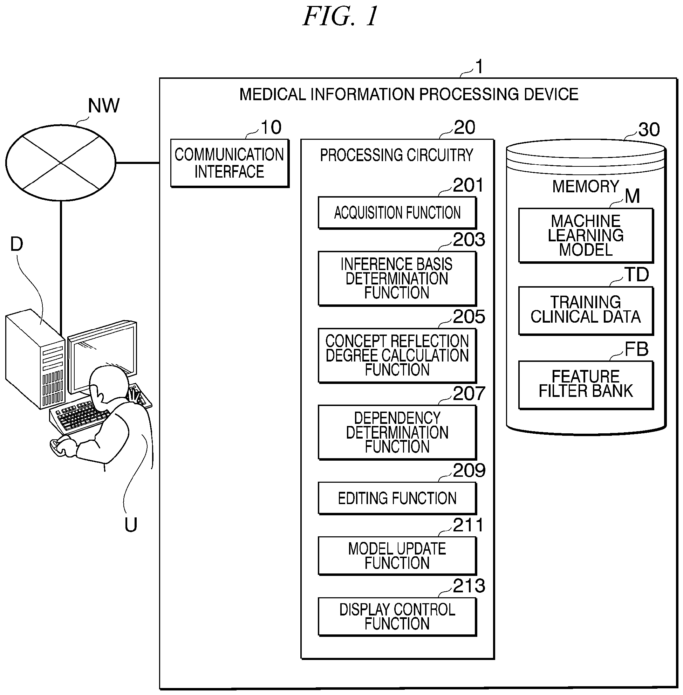

FIG. 1 is a functional block diagram showing an example of a configuration of a medical information processing device 1 according to an embodiment. The medical information processing device 1 is connected to at least one or more terminal devices D via a communication network NW such that they can communicate with each other. The communication network NW refers to a general information and communication network that uses electrical communication technology. For example, the communication network NW includes wireless/wired local area networks (LANs) such as hospital backbone LANs and Internet networks, telephone communication line networks, optical fiber communication networks, cable communication networks, satellite communication networks, and the like. The medical information processing device 1 is an example of a "medical information processing device."

The terminal device D is operated by, for example, a doctor U (user) who checks various types of information related to a machine learning model and controls training of the machine learning model. The terminal device D is, for example, a personal computer, a mobile terminal such as a tablet or a smartphone, or the like. The terminal device D has a display function such as a liquid crystal display that displays various types of information, and an input function that is an input interface for receiving various input operations by the doctor U. The input interface is realized by a mouse, a keyboard, a touch panel, a track ball, a switch, buttons, a joystick, a camera, an infrared sensor, a microphone, etc. In this specification, the input interface is not limited to interfaces having physical operation parts such as a mouse and a keyboard. Examples of the input interface also include an electrical signal processing circuit that receives an electrical signal corresponding to an input operation from external input equipment provided separately from the device and outputs this electrical signal to a control circuit, for example. Terminal device D is an example of a "terminal device."

The medical information processing device 1 includes, for example, a communication interface 10, processing circuitry 20, and a memory 30. The communication interface 10 communicates with an external device via a communication network NW. The communication interface 10 includes, for example, a network interface card (NIC) and an antenna for wireless communication. The memory 30 stores a machine learning model M, training clinical data TD (training data), a feature filter bank FB, and the like. The memory 30 is realized, for example, by a semiconductor memory element such as a random access memory (RAM) or a flash memory, a hard disk, an optical disc, or the like. The machine learning model M, the training clinical data TD, and the feature filter bank FB may be stored in an external memory with which the medical information processing device 1 can communicate, instead of the memory 30 (or in addition to the memory 30). The external memory is controlled by a cloud server that manages the external memory, for example, by the cloud server receiving a read/write request.

The machine learning model M is a model trained to perform desired classification processing and the like according to a purpose. The machine learning model M is a model that performs, for example, skin disease classification processing, cell image classification processing, and the like. The machine learning model M is generated using any machine learning method such as a neural network, a support vector machine, a decision tree, and the like. Neural networks include, for example, a convolutional neural network (CNN), a recurrent neural network (RNN), an autoencoder, and the like.

The training clinical data TD is training data used in a training stage of the machine learning model M. The training clinical data TD is image data or non-image data. Image data includes, for example, magnetic resonance (MR) images, computed tomography (CT) images, images captured by imaging devices such as cameras and microscopes (for example, skin images and cell images), and the like. Non-image data includes, for example, data on results of specimen tests, vital data measured by electrocardiographs, pulse meters, and the like, attribute information of subjects, and the like.

The processing circuitry 20 controls the overall operation of the medical information processing device 1. The processing circuitry 20 executes, for example, an acquisition function 201, an inference basis determination function 203, a concept reflection degree calculation function 205, a dependency determination function 207, an editing function 209, a model update function 211, and a display control function 213. These functions are realized, for example, by a hardware processor (computer) executing a program (software) stored in the memory 30. The hardware processor is, for example, circuitry such as a central processing unit (CPU), a graphics processing unit (GPU), a system on chip (SOC), an application specific integrated circuit (ASIC), or a programmable logic device (for example, a simple programmable logic device (SPLD) or a complex programmable logic device (CPLD), or a field programmable gate array (FPGA)). Instead of storing the program in the memory 30, the program may be directly embedded in the circuit of the hardware processor. In this case, the hardware processor realizes a function by reading and executing the program embedded in the circuit. The hardware processor is not limited to being configured as a single circuit, but may be configured as one hardware processor by combining a plurality of independent circuits to realize each function. Further, each function may be realized by integrating a plurality of components into one hardware processor.

The acquisition function 201 acquires the machine learning model M stored in the memory 30, the training clinical data TD used for training the machine learning model M, various types of information transmitted from the terminal device D, and the like. The acquisition function 201 is an example of an "acquirer."

The inference basis determination function 203 uses the machine learning model M to determine an inference basis of each piece of the training clinical data TD and generates an inference basis visualization result. The inference basis determination function 203 generates, for example, an inference basis map. The inference basis determination function 203 uses, for example, a Grad-CAM method to determine the inference basis for the training clinical data TD, which is image data, and generates an inference basis visualization result (inference basis map). Grad-CAM is a method of visualizing what the machine learning model M configured by a CNN focuses on in the image data (i.e., the inference basis). In addition, the inference basis determination function 203 uses a Shapley Additive Explanations (SHAP) method to determine the inference basis for the training clinical data TD, which is non-image data, and generates an inference basis visualization result. SHAP is a method of calculating the contribution of each feature to an inference result of a machine learning model (the influence of an increase or decrease in the value of a variable of a feature on an inference result). The inference basis determination function 203 is an example of an "inference basis determiner."

The concept reflection degree calculation function 205 determines a concept emphasized by the machine learning model M during inference on the basis of inference basis visualization results, and calculates a concept reflection degree of each piece of the training clinical data TD with respect to the concept. As a first procedure, the concept reflection degree calculation function 205 extracts a main inference basis visualization result. For example, the concept reflection degree calculation function 205 clusters inference basis maps on the basis of a similarity in the appearance of the inference basis maps, and calculates the centroid of each cluster. Clustering includes, for example, methods such as k-means and hierarchical clustering. The similarity includes, for example, a cosine similarity of Fourier transform results (in the case of image data), a cosine similarity (in the case of non-image data), and the like. As a second procedure, the concept reflection degree calculation function 205 calculates a concept reflection degree of each piece of the training clinical data TD. For example, the concept reflection degree calculation function 205 calculates a concept reflection degree of a main concept for each piece of the training clinical data TD on the basis of the distance from a cluster centroid of clustered concepts. The concept reflection degree of the training clinical data TD is calculated such that the concept reflection degree increases as the distance from the cluster centroid of clustered concepts decreases and decreases as the distance increases. The concept reflection degree calculation function 205 is an example of a "concept reflection degree calculator."

The dependency determination function 207 generates visualization information of dependency between a concept and an interpretation support feature on the basis of the concept reflection degree and features that can be interpreted by a doctor (user) in the training clinical data TD (hereinafter referred to as interpretation support features"). For example, the dependency determination function 207 uses the calculated concept reflection degree for each piece of training clinical data TD as an objective variable, uses a predefined interpretation support feature calculated from each piece of training clinical data TD as an explanatory variable, determines the dependency therebetween, and generates a dependency graph, which is visualization information, by graphical modeling. The dependency determination function 207 is an example of a "dependency determiner."

Graphical modeling includes, for example, Markov Random Field (MRF) using an undirected graph expressing correlation, Bayesian network using a directed graph expressing causal relationship, and the like. Interpretation support features calculated from the training clinical data TD are features associated with a certain concept and converted into information that is easy for a doctor to interpret. When the training clinical data TD is image data, interpretation support features include, for example, at least one of color features (red component and brown component), features related to texture (uniformity, frequency of occurrence of elliptical or circular areas), and features related to shapes (complexity of an outline for a binarized result and circularity). When the training clinical data TD is non-image data, interpretation support features include, for example, features calculated on the basis of a predetermined clinical guideline. Features calculated on the basis of the clinical guideline include, for example, an acute physiology and chronic health evaluation (APACHE) score (a score of the severity of a patient admitted to an ICU based on values such as respiration, circulation, and blood test values). A function for expressing each interpretation support feature is defined in advance and registered in the feature filter bank FB stored in the memory 30. Alternatively, the doctor U may create and set their own features.

The editing function 209 edits the visualization information (dependency graph) generated by the dependency determination function 207 in accordance with an instruction of the doctor U (user) to match the intuition (clinical knowledge) of the doctor. Editing includes, for example, node addition, node deletion, edge editing, and the like. Node addition is adding an interpretation support feature that matches the intuition of the doctor, selected from the interpretation support features registered in the feature filter bank FB, to the dependency graph. Node deletion is deleting an interpretation support feature that does not match the intuition of the doctor from the dependency graph. Edge editing is defining the strength of a dependency in the dependency graph. In the case of a feature that is difficult to calculate using existing features, it may be possible to create the same using machine learning techniques. Learning is performed using deep distance learning such that a target image and a non-target image are distinguished from each other to train a feature extractor capable of quantifying the reflection degree of the target image. After learning, results are newly added to the feature filter bank FB. For example, in a case where similar concepts are to be intentionally separated, this can be achieved through node addition or edge editing. For example, in a case where erythema is emphasized and capillary dilation is not emphasized, adjustment can be performed such that only erythema is emphasized among concepts having the same red characteristic by setting a round to a positive correlation and contour complexity to a negative correlation. The editing function 209 is an example of an "editor."

The model update function 211 performs additional training of the machine learning model M on the basis of the visualization information edited by the editing function 209 (the dependency graph edited to match the intuition of the doctor) to update the machine learning model M. The model update function 211 performs additional training of the machine learning model M such that a dependency derived from the machine learning model M matches a dependency corresponding to the edited visualization information. For example, a loss function is defined as the sum of a classification error and an error between a model-derived dependency and a dependency defined by the doctor, and additional training is performed to change the weight of the machine learning model M such that this loss function becomes smaller. The model update function 211 is an example of a "model updater."

The display control function 213 displays the visualization information (dependency graph) generated by the dependency determination function 207, a graphical user interface (GUI) image for receiving various input operations by the doctor U, such as an instruction to edit the machine learning model M, and the like, on a display device of the terminal device D. The display control function 213 is an example of a "display controller."

[Processing Flow]

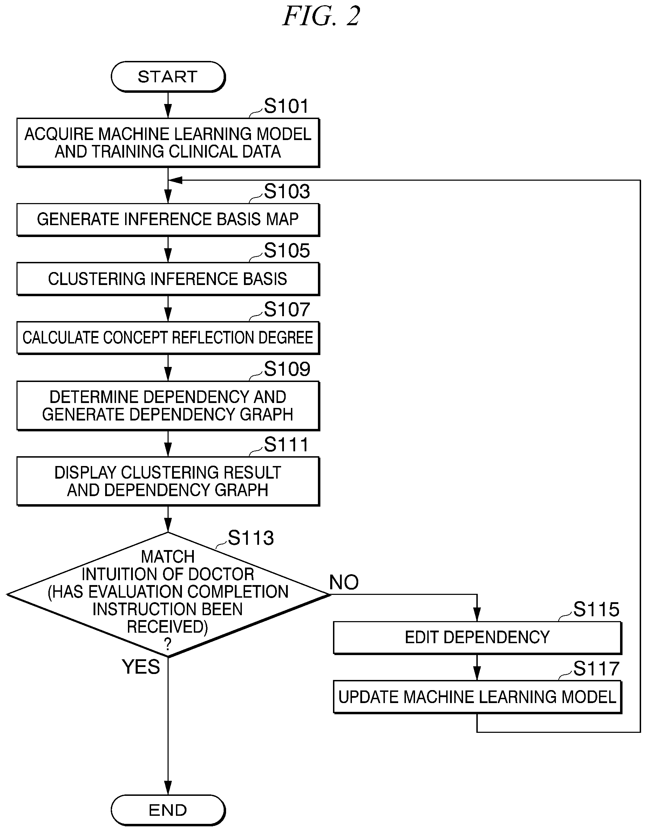

Next, a flow of processing of the medical information processing device 1 will be described. FIG. 2 is a flowchart showing an example of processing of the medical information processing device 1 according to the embodiment. Processing shown in FIG. 2 is executed, for example, before product development and operation after proof of concept (PoC) of the machine learning model M is implemented, when a trained machine learning model M (e.g., a skin cancer classification model) is processed to emphasize clinically valid information. Processing shown in FIG. 2 is started, for example, when the doctor U inputs an instruction to start processing via an input interface of the terminal device D. In the following, an example will be described in which the machine learning model M is a skin cancer classification model and the training clinical data TD is image data.

First, the acquisition function 201 acquires, from the memory 30, the machine learning model M to be evaluated and the training clinical data TD used to train the machine learning model M (step S101). This training clinical data TD is, for example, a mixture of training data to which a ground truth label of "presence of cancer" or "absence of cancer" in skin cancer classification has been assigned.

Next, the inference basis determination function 203 uses the machine learning model M to determine the inference basis of each piece of the training clinical data TD and generates an inference basis map (step S103). The inference basis determination function 203 generates an inference basis map for each piece of the training clinical data TD, for example, using a Grad-CAM method. For example, if the training clinical data TD is 100 pieces of image data, the inference basis determination function 203 generates 100 inference basis maps.

Next, the concept reflection degree calculation function 205 clusters the inference basis maps depending on a similarity in the appearance of the inference basis maps, and calculates the centroid of each cluster (step S105). For example, the concept reflection degree calculation function 205 clusters the 100 inference basis maps using a method such as k-means or hierarchical clustering on the basis of the cosine similarity of Fourier transform results of each of the 100 inference basis maps.

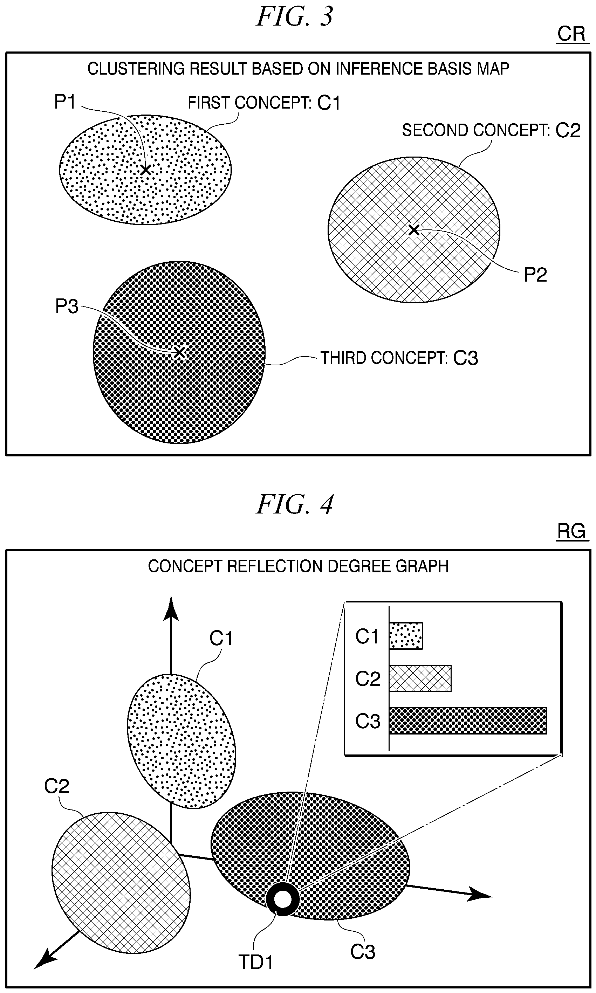

FIG. 3 is a diagram showing an example of a clustering result CR based on inference basis maps according to the embodiment. In FIG. 3, an example is shown in which a first concept C 1 (centroid P1), a second concept C2 (centroid P2), and a third concept C3 (centroid P3) are calculated as main concepts as representative concepts.

Next, the concept reflection degree calculation function 205 calculates a concept reflection degree of each piece of the training clinical data TD (step S107). For example, the concept reflection degree calculation function 205 calculates a main concept reflection degree for each piece of the training clinical data TD from a distance to the centroid of each cluster (concept). The concept reflection degree of the training clinical data TD is calculated such that it increases as the centroid of each cluster decreases and decreases as the centroid of each cluster increases.

FIG. 4 is a diagram showing an example of a concept reflection degree RG for each piece of training clinical data according to the embodiment. In FIG. 4, the first concept C1, the second concept C2, and the third concept C3 calculated in FIG. 3 are defined on three axes, and the concept reflection degree RG for each piece of training clinical data TD is indicated on a graph. On this graph, for example, training clinical data TD1, which is a certain piece of image data, is closest to the centroid P3 of the third concept C3, and is further away from the centroid P2 of the second concept C2 and the centroid P1 of the first concept C1 in that order. That is, the magnitude of the concept reflection degree of the training clinical data TD 1 is in the order of the third concept, the second concept, and the first concept.

Referring back to FIG. 2, next, the dependency determination function 207 determines a dependency between each of the representative concepts determined from the training clinical data TD and features (interpretation support features) extracted from the training clinical data TD, and generates a dependency graph (step S109).

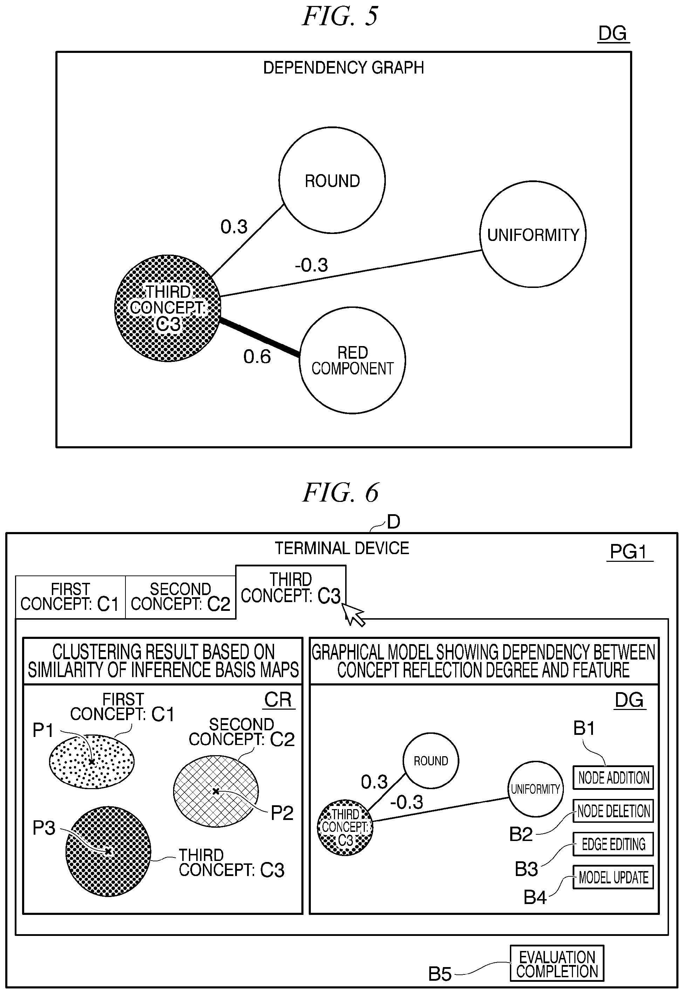

FIG. 5 is a diagram showing an example of a dependency graph DG according to the embodiment. In FIG. 5, a dependency between the third concept C3 (node destination) and each of interpretation support features of "round," "uniformity," and "red component" (node source) extracted from the training clinical data TD is represented by a Markov Random Field (MRF). A numerical value (correlation coefficient) indicating the strength of connection between the node destination and the node source is attached to the edge connecting the node destination and the node source. The example of FIG. 5 shows that the dependency between the third concept and "red component" is the highest.

Referring back to FIG. 2, next, the display control function 213 causes the terminal device D to display a screen showing the clustering result and the dependency graph (step S111).

FIG. 6 is a diagram showing an example of a first screen PG1 including a clustering result CR and a dependency graph DG displayed on the terminal device D according to the embodiment. By checking the first screen PG1 displayed on the terminal device D, the doctor U can ascertain the dependency between concepts emphasized in the inference in the machine learning model M and interpretation support features associated with each concept and check whether the concepts match the intuition of the doctor U.

Referring back to FIG. 2, next, the acquisition function 201 determines whether dependencies matches the intuition of the doctor U (whether an evaluation completion instruction has been received from the terminal device D) (step S113). When the doctor U who has checked the first screen PG1 displayed on the terminal device D thinks that the concepts match the intuition of the doctor U (i.e., thinks that the concepts of the machine learning model M are appropriate and additional training is unnecessary) and presses an evaluation completion button B5, the acquisition function 201 acquires an evaluation completion instruction (step S 113 : YES), and processing of this flowchart ends.

On the other hand, if the doctor U who has checked the first screen PG1 displayed on the terminal device D thinks that the concepts do not match the intuition of the doctor U (i.e., thinks that the concepts of the machine learning model M are inappropriate and additional training is necessary) and does not press the evaluation completion button B5, and the acquisition function 201 receives an editing instruction without acquiring an evaluation completion instruction (step S 113 : NO), the editing function 209 edits the dependencies in accordance with the received editing instruction (step S 115). Editing includes, for example, node addition, node deletion, edge editing, and the like.

(Node Addition)

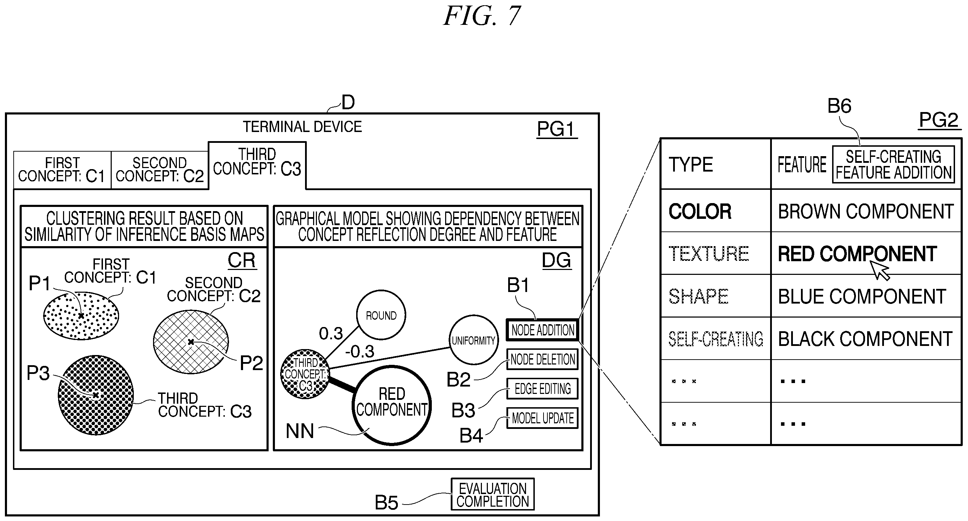

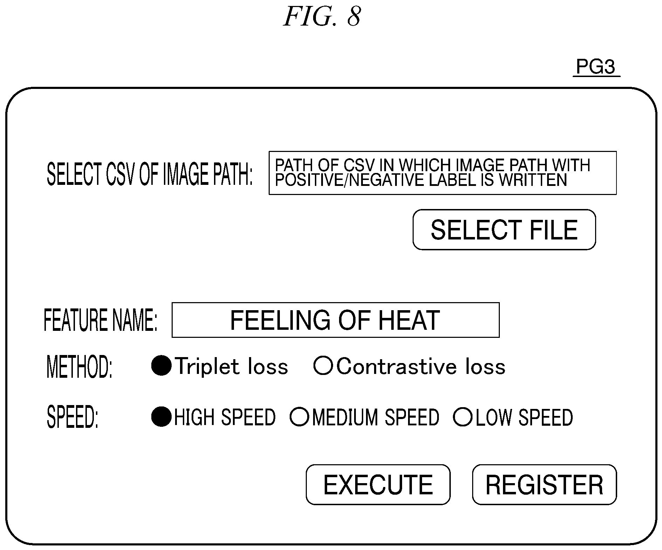

FIG. 7 is a diagram showing an example of a screen display at the time of performing editing processing of adding a node according to the embodiment. When the doctor U who has checked the first screen PG1 displayed on the terminal device D presses a node addition button B 1 by operating the input interface, a second screen PG2 for designating an interpretation support feature to be added as a node is displayed. The second screen PG2 lists interpretation support features that are predefined and registered in the feature filter bank FB stored in the memory 30, and the doctor U can select and add a desired interpretation support feature. In the example of FIG. 7, the feature "red component" is selected, the dependency graph DG of the first screen PG1 is edited, and a node NN of "red component" is added. When the doctor U presses a self-creating feature addition button B6 provided on the second screen PG2 by operating the input interface, a third screen PG3 for self-creating and adding an interpretation support feature as shown in FIG. 8 is displayed. Through this third screen PG3, the doctor U can input information on the interpretation support feature they wish to add to newly register a definition of the interpretation support feature in the feature filter bank FB and add the same to the dependency graph DG.

(Node Deletion)

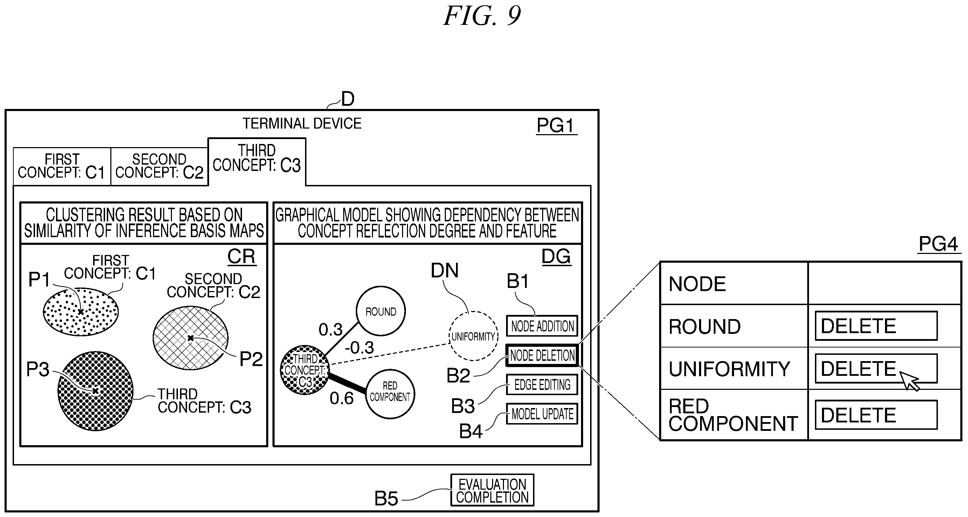

FIG. 9 is a diagram showing an example of a screen display at the time of performing editing processing for node deletion according to the embodiment. When the doctor U who has checked the first screen PG1 displayed on the terminal device D presses a node deletion button B2 by operating the input interface, a fourth screen PG4 for designating a node to be deleted is displayed. The fourth screen PG4 lists interpretation support features set as nodes in the dependency graph DG of the first screen PG1, and the doctor U can select and delete an interpretation support feature they wish to delete. In the example of FIG. 9, the feature "uniformity" is selected, the dependency graph DG of the first screen PG1 is edited, and a node DN of "uniformity" is deleted.

(Edge Editing)

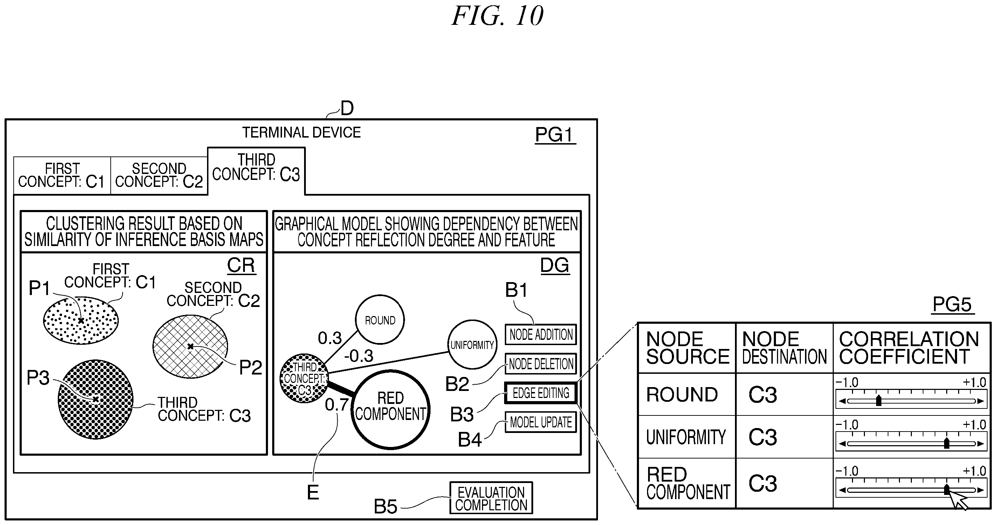

FIG. 10 is a diagram showing an example of a screen display at the time of performing editing processing for edge editing according to the embodiment. When the doctor U who has checked the first screen PG1 displayed on the terminal device D presses an edge editing button B3 by operating the input interface, a fifth screen PG5 for designating an edge to be edited is displayed. The fifth screen PG5 lists edges indicated by a node source and a node destination set in the dependency graph DG on the first screen PG1, and the doctor U can designate the numerical value of a correlation coefficient of the edge they want to edit (for example, by moving a slider) to edit the edge. In the example of FIG. 10, an edge whose node source is "red component" is selected, the numerical value of the correlation coefficient is changed to "0.7," and the dependency graph DG of the first screen PG1 is edited to change the numerical value of the correlation coefficient of edge E to "0.7."

Referring back to FIG. 2, after the editing function 209 has edited dependencies, when the doctor U operates the input interface to press a model update button B4 set on the first screen PG1, the model update function 211 performs additional training of the machine learning model M on the basis of the dependencies edited by the editing function 209 (dependencies edited to match the intuition of the doctor) to update the machine learning model M (step S117).

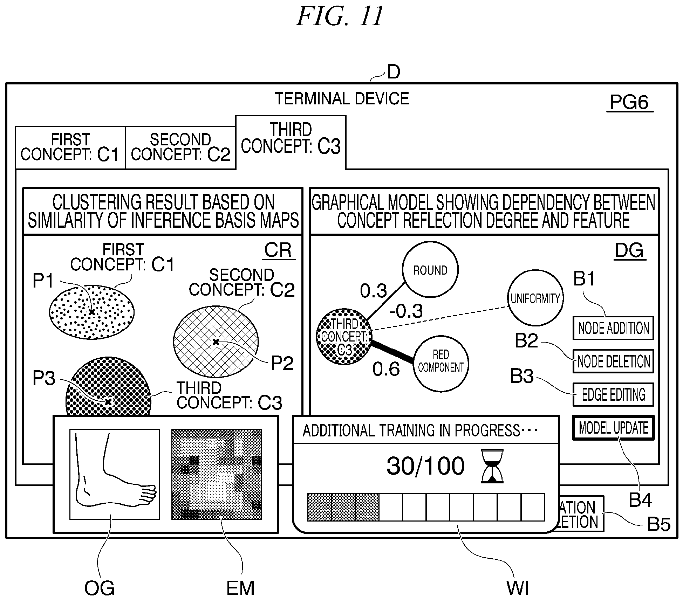

FIG. 11 is a diagram showing an example of a sixth screen PG6 that is displayed during update of the machine learning model according to the embodiment. This sixth screen PG6 displays a display W1 indicating that additional training is being performed. After additional training is completed, when a cursor is placed on each concept displayed in the clustering result CR included in the sixth screen PG6, an original image OG and an inference basis map EM are enlarged and displayed.

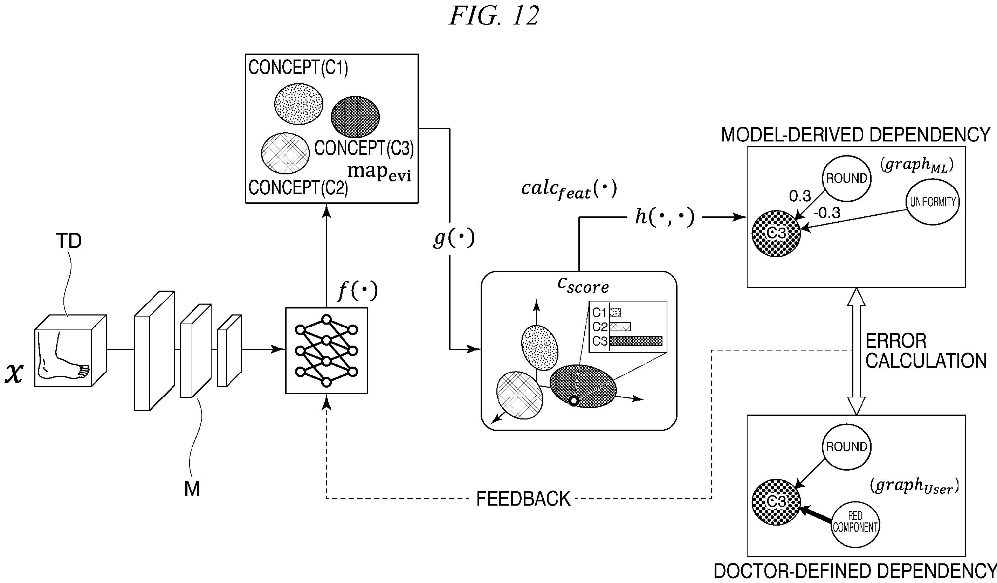

FIG. 12 is a diagram showing an example of a flow of model update processing according to the embodiment. The model update function 211 performs additional training of the machine learning model M such that a dependency derived from the machine learning model M matches a dependency edited to match the intuition of the doctor. For example, a loss function is defined as the sum of a classification error and an error between a dependency derived from the model and a dependency defined by the doctor, and additional training is performed to change the weight of the machine learning model M such that this loss function becomes smaller. As an example of the error, a similarity between a dependency between a concept and a feature based on the output of the machine learning model M (model-derived dependency, graphML) and a dependency between a concept and a feature defined by the doctor (doctor-defined dependency, graphUser) is used. The definition of each formula shown in FIG. 12 is as follows. As an example of a method of calculating the similarity, the graph edit distance is used.

[Expression 1]

$$x: \text{Training data} \in \mathbf{R}^{d \times n}$$

$$d: \text{Number of input dimensions, n: total number of pieces of training data}$$

[Expression 2]

$$f(\cdot): \text{Model inference basis calculation processing}$$

$$\text{map}_{evi} = f(x), \text{map}_{evi} \in R^{d \times n}$$

[Expression 3]

$$g(\cdot): \text{Processing of identifying concept through comprehensive determination}$$

$$\text{from inference basis calculation processing and calculating concept reflection degree}$$

$$c_{score} = g(\text{map}_{evi}), c_{score} \in R^{C \times n}, C: \text{total number of concepts}$$

[Expression 4]

$$h(\cdot, \cdot): \text{Processing of making relationship between concept reflection degree and}$$

$$\text{features of training data into graph}$$

$$graph_{ML} = h(calc_{feat}(x), c_{score})$$

$calc_{feat}(\cdot)$: Processing of calculating predefined feature that is easy to interpret for training data

Referring back to FIG. 2, after update of the machine learning model M is completed, processing returns to step S103, in which the inference basis of each piece of the training clinical data TD is determined again using the updated machine learning model M, an inference basis map is generated, and subsequent processing is repeated. Finally, when an evaluation completion instruction is received (step S 113 : YES), processing of this flowchart ends.

According to the embodiment described above, by providing information indicating the relationship (dependency) between information (concept) emphasized by a machine learning model during inference and a feature calculated from training data, it is possible to confirm the clinical validity of the inference basis of the machine learning model. Furthermore, by updating the machine learning model to match a dependency edited according to an instruction of an operator (doctor), it is possible to generate a machine learning model that can operate with an inference basis corresponding to the intuition (clinical knowledge) of the doctor, and thus improve the accuracy of the machine learning model. This makes it possible to prevent a doctor from referring to inference results of a model influenced by confounding factors when using the machine learning model for clinical doctor decision support.

Furthermore, each function of the medical information processing device 1 described in the above embodiment may be realized by installing an application in the terminal device D. In this case, the terminal device D is an example of a "medical information processing device."

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims.

## Claims

1.   A medical information processing device comprising:

   an acquirer configured to acquire a machine learning model and training data used to train the machine learning model;
   an inference basis determiner configured to determine an inference basis for each piece of the training data using the machine learning model to generate inference basis visualization results;
   a concept reflection degree calculator configured to determine a concept emphasized by the machine learning model during inference based on the inference basis visualization results and calculate a concept reflection degree of each piece of the training data related to the concept; and
   a dependency determiner configured to generate visualization information of a dependency between the concept and a feature interpretable by a user in the training data based on the concept reflection degree and the feature interpretable by the user.

2.   The medical information processing device according to claim 1, further comprising:

   an editor configured to edit the visualization information in response to an instruction from the user; and
   a model updater configured to update the machine learning model based on the edited visualization information.

3.   The medical information processing device according to claim 1 or 2, wherein the dependency determiner is configured to generate a dependency graph, which is the visualization information, by graphical modeling.

4.   The medical information processing device according to claim 1 or 2, further comprising a display controller configured to cause a display device to display the visualization information.

5.   The medical information processing device according to claim 2, wherein the model updater is configured to additionally train the machine learning model to match the dependency derived from the machine learning model with a dependency corresponding to the edited visualization information.

6.   The medical information processing device according to claim 1 or 2, wherein the concept reflection degree calculator is configured to:

   determine the concept by clustering the inference basis visualization results based on a similarity of the inference basis visualization results; and

calculate the concept reflection degree of each piece of the training data based on a distance from a cluster centroid of the clustered concept.

7. The medical information processing device according to claim 6, wherein the concept reflection degree calculator is configured to calculate the concept reflection degree such that the concept reflection degree increases as the distance from the cluster centroid of the clustered concept decreases and decreases as the distance increases.

8. The medical information processing device according to claim 1 or 2, wherein, when the training data is image data, the feature interpretable by the user includes at least one of a feature with respect to a color, a feature with respect to texture, and a feature with respect to a shape.

9. The medical information processing device according to claim 1 or 2, wherein, when the training data is non-image data, the feature interpretable by the user includes features calculated based on a predetermined guideline.

10. The medical information processing device according to claim 2, wherein the editor is configured to add the interpretable feature designated by the user to the visualization information.

11. The medical information processing device according to claim 2, wherein the editor is configured to delete the interpretable feature designated by the user from the visualization information.

12. The medical information processing device according to claim 2, wherein the editor is configured to newly add a definition of the interpretable feature based on an instruction from the user.

13. The medical information processing device according to claim 1 or 2, wherein the feature interpretable by the user is predefined.

14. A medical information processing method, using a computer, comprising:

acquiring a machine learning model and training data used to train the machine learning model;
determining an inference basis for each piece of the training data using the machine learning model to generate inference basis visualization results;
determining a concept emphasized by the machine learning model during inference based on the inference basis visualization results and calculating a concept reflection degree of each piece of the training data related to the concept; and
generating visualization information of a dependency between the concept and a feature interpretable by a user in the training data based on the concept reflection degree and the feature interpretable by the user.

15. A program causing a computer to:

acquire a machine learning model and training data used to train the machine learning model;
determine an inference basis for each piece of the training data using the machine learning model to generate inference basis visualization results;
determine a concept emphasized by the machine learning model during inference based on the inference basis visualization results and calculate a concept reflection degree of each piece of the training data related to the concept; and
generate visualization information of a dependency between the concept and a feature interpretable by a user in the training data based on the concept reflection degree and the feature interpretable by the user.

*FIG. 1*

*FIG. 2*

```
                    ┌─────────────┐
                    │    START    │
                    └──────┬──────┘
                           │        S101
           ┌───────────────▼────────────────┐
           │ ACQUIRE MACHINE LEARNING MODEL  │
           │   AND TRAINING CLINICAL DATA    │
           └───────────────┬────────────────┘
                           │        S103
           ┌───────────────▼────────────────┐
           │  GENERATE INFERENCE BASIS MAP   │
           └───────────────┬────────────────┘
                           │        S105
           ┌───────────────▼────────────────┐
           │   CLUSTERING INFERENCE BASIS    │
           └───────────────┬────────────────┘
                           │        S107
           ┌───────────────▼────────────────┐
           │ CALCULATE CONCEPT REFLECTION DEGREE │
           └───────────────┬────────────────┘
                           │        S109
           ┌───────────────▼────────────────┐
           │   DETERMINE DEPENDENCY AND      │
           │  GENERATE DEPENDENCY GRAPH      │
           └───────────────┬────────────────┘
                           │        S111
           ┌───────────────▼────────────────┐
           │   DISPLAY CLUSTERING RESULT     │
           │    AND DEPENDENCY GRAPH         │
           └───────────────┬────────────────┘
                           │        S113
                      ╱────▼────╲
                    ╱   MATCH     ╲
                  ╱ INTUITION OF    ╲   NO      S115
                 ╱  DOCTOR (HAS      ╲──────► ┌──────────────────┐
                ╲  EVALUATION        ╱        │ EDIT DEPENDENCY  │
                 ╲ COMPLETION       ╱         └────────┬─────────┘
                  ╲ INSTRUCTION    ╱                   │    S117
                    ╲ BEEN        ╱          ┌──────────▼───────────────┐
                     ╲RECEIVED)? ╱           │ UPDATE MACHINE LEARNING  │
                      ╲────┬────╱            │         MODEL            │
                        YES│                 └──────────────────────────┘
                    ┌──────▼──────┐
                    │     END     │
                    └─────────────┘
```

*FIG. 3*

<u>CR</u>

CLUSTERING RESULT BASED ON INFERENCE BASIS MAP

P1 — FIRST CONCEPT: C1

SECOND CONCEPT: C2

P2

P3 — THIRD CONCEPT: C3

*FIG. 4*

<u>RG</u>

CONCEPT REFLECTION DEGREE GRAPH

C1

C2

C1

C2

C3

TD1 — C3

## FIG. 5

DG

DEPENDENCY GRAPH

ROUND

UNIFORMITY

0.3

-0.3

THIRD CONCEPT: C3

0.6

RED COMPONENT

## FIG. 6

D

TERMINAL DEVICE

PG1

FIRST CONCEPT: C1

SECOND CONCEPT: C2

THIRD CONCEPT: C3

CLUSTERING RESULT BASED ON SIMILARITY OF INFERENCE BASIS MAPS

CR

FIRST CONCEPT: C1

P1

SECOND CONCEPT: C2

P2

P3

THIRD CONCEPT: C3

GRAPHICAL MODEL SHOWING DEPENDENCY BETWEEN CONCEPT REFLECTION DEGREE AND FEATURE

DG

B1

ROUND

UNIFORMITY

0.3

-0.3

THIRD CONCEPT: C3

NODE ADDITION

NODE DELETION

B2

EDGE EDITING

B3

MODEL UPDATE

B4

B5

EVALUATION COMPLETION

## FIG. 7

TERMINAL DEVICE — D — PG1

FIRST CONCEPT: C1 | SECOND CONCEPT: C2 | THIRD CONCEPT: C3

**CLUSTERING RESULT BASED ON SIMILARITY OF INFERENCE BASIS MAPS** — CR

FIRST CONCEPT: C1 — P1
SECOND CONCEPT: C2 — P2
THIRD CONCEPT: C3 — P3

**GRAPHICAL MODEL SHOWING DEPENDENCY BETWEEN CONCEPT REFLECTION DEGREE AND FEATURE** — DG

B1 — NODE ADDITION
ROUND
0.3
-0.3
THIRD CONCEPT: C3
UNIFORMITY
RED COMPONENT
NN
NODE DELETION — B2
EDGE EDITING — B3
MODEL UPDATE — B4

EVALUATION COMPLETION — B5

B6 — PG2

| TYPE | FEATURE | SELF-CREATING FEATURE ADDITION |
|------|---------|-------------------------------|
| **COLOR** | BROWN COMPONENT | |
| TEXTURE | **RED COMPONENT** | |
| SHAPE | BLUE COMPONENT | |
| SELF-CREATING | BLACK COMPONENT | |
| . . . | . . . | |
| . . . | . . . | |

EP 4 586 156 A1

*FIG. 8*

PG3

SELECT CSV OF IMAGE PATH: | PATH OF CSV IN WHICH IMAGE PATH WITH POSITIVE/NEGATIVE LABEL IS WRITTEN

SELECT FILE

FEATURE NAME: | FEELING OF HEAT

METHOD: ● Triplet loss ○ Contrastive loss

SPEED: ● HIGH SPEED ○ MEDIUM SPEED ○ LOW SPEED

EXECUTE | REGISTER

## FIG. 9

TERMINAL DEVICE — D — PG1

FIRST CONCEPT: C1 | SECOND CONCEPT: C2 | THIRD CONCEPT: C3

CLUSTERING RESULT BASED ON SIMILARITY OF INFERENCE BASIS MAPS — CR

- FIRST CONCEPT: C1 — P1
- SECOND CONCEPT: C2 — P2
- THIRD CONCEPT: C3 — P3

GRAPHICAL MODEL SHOWING DEPENDENCY BETWEEN CONCEPT REFLECTION DEGREE AND FEATURE — DG

DN

ROUND
0.3
-0.3
UNIFORMITY
THIRD CONCEPT: C3
0.6
RED COMPONENT

NODE ADDITION — B1
NODE DELETION — B2
EDGE EDITING — B3
MODEL UPDATE — B4

EVALUATION COMPLETION — B5

PG4

| NODE | |
|---|---|
| ROUND | DELETE |
| UNIFORMITY | DELETE |
| RED COMPONENT | DELETE |

EP 4 586 156 A1

*FIG. 10*

## FIG. 11

D

**TERMINAL DEVICE** — PG6

| FIRST CONCEPT: C1 | SECOND CONCEPT: C2 | THIRD CONCEPT: C3 |

**CLUSTERING RESULT BASED ON SIMILARITY OF INFERENCE BASIS MAPS** — CR

FIRST CONCEPT: C1
P1
SECOND CONCEPT: C2
P3
P2
THIRD CONCEPT: C3

OG    EM

**GRAPHICAL MODEL SHOWING DEPENDENCY BETWEEN CONCEPT REFLECTION DEGREE AND FEATURE** — DG

ROUND
0.3
-0.3
UNIFORMITY
B1
THIRD CONCEPT: C3
RED COMPONENT
0.6

NODE ADDITION
B2
NODE DELETION
B3
EDGE EDITING
MODEL UPDATE
B4

ADDITIONAL TRAINING IN PROGRESS···

30/100 ⌛

...ATION ...ETION — B5

WI

# FIG. 12

Europäisches
Patentamt

European
Patent Office

Office européen
des brevets

# EUROPEAN SEARCH REPORT

**Application Number**

EP 25 15 0727

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SIYUAN YAN ET AL: "Towards Trustable Skin Cancer Diagnosis via Rewriting Model's Decision", ARXIV.ORG, CORNELL UNIVERSITY LIBRARY, 201 OLIN LIBRARY CORNELL UNIVERSITY ITHACA, NY 14853, 2 March 2023 (2023-03-02), XP091450289, * page 1 - page 8 * * Algorithm 1; page 13 - page 17; figures 1-17; tables 4-6 * | 1-15 | INV. G06N20/00 G06N7/01 ADD. G06N5/045 |

- - - - -

**TECHNICAL FIELDS SEARCHED (IPC)**

G06N

The present search report has been drawn up for all claims

| Place of search | Date of completion of the search | Examiner |
|---|---|---|
| The Hague | 21 May 2025 | Cilia, Elisa |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...........................................................................
& : member of the same patent family, corresponding
   document

EPO FORM 1503 03.82 (P04C01)